Europäisches Patentamt

European Patent Office

Office européen des brevets

(19)

(11) Veröffentlichungsnummer: **0 237 786**
**B1**

(12) **EUROPÄISCHE PATENTSCHRIFT**

(45) Veröffentlichungstag der Patentschrift:
19.12.90

(51) Int. Cl.⁵: **A61F 13/15**

(21) Anmeldenummer: **87101944.4**

(22) Anmeldetag: **12.02.87**

(54) **Hygienischer Zellstoffartikel mit Haftklebeschicht.**

(30) Priorität: **22.02.86 DE 3605842**

(43) Veröffentlichungstag der Anmeldung:
**23.09.87 Patentblatt 87/39**

(45) Bekanntmachung des Hinweises auf die Patenterteilung:
**19.12.90 Patentblatt 90/51**

(84) Benannte Vertragsstaaten:
**AT BE CH FR GB IT LI NL SE**

(56) Entgegenhaltungen:
**CH-A- 470 525**
**DE-U- 8 522 762**
**GB-A- 781 975**
**GB-A- 789 890**
**GB-A- 2 081 101**

(73) Patentinhaber: **VP - SCHICKEDANZ AG,**
**Schoppershofstrasse 80, D-8500 Nürnberg 1(DE)**

(72) Erfinder: **Haegler, Walter, Dr., Holbeinstrasse 5,**
**D-8507 Oberasbach(DE)**
Erfinder: **Hübner, Peter, Waldstrasse 7,**
**D-8501 Winkelhaid(DE)**
Erfinder: **Petranyi, Paul, Dr., Schwerinerstrasse 15,**
**D-8500 Nürnberg(DE)**

(74) Vertreter: **Pohl, Hans Ludwig, Hefnersplatz 3,**
**D-8500 Nürnberg 11(DE)**

## Beschreibung

Die Erfindung betrifft einen hygienischen Zellstoffartikel gemäß dem Oberbegriff des Anspruchs 1. Ein derartiger Zellstoffartikel ist aus der DE-U 8 522 762 bekannt. Derartige hygienische Zellstoffartikel finden als Damenbinden, Slipeinlagen oder dergl. vielfältige Verwendung. Sie sind in zahlreichen Druckschriften beschrieben, beispielsweise in der DE-A 3 128 398.

Bei hygienischen Zellstoffartikeln der genannten Art besteht das Problem, die Haftklebeschicht an der Unterseite des Artikels so auszugestalten, daß einerseits eine sichere Befestigung im Kleidungsstück möglich ist, die auch bei starker Bewegung des Trägers Verrutschen und Ablösen verhindert, die aber andererseits so ausgestaltet ist, daß beim gewollten Ablösen des Zellstoffartikels weder Klebstoff im Kleidungsstück zurückbleibt, noch Fasern aus dem Kleidungsstück herausgelöst werden. Zur Lösung des an erster Stelle genannten Teilproblems, also dem möglichst rutschfesten Sichern des Zellstoffartikels im Kleidungsstück wird in der DE-A 3 128 398 vorgeschlagen, den Kleber in einem ungeordneten Muster an der dem Kleidungsstück zugewandten Seite der undurchlässigen Sperrlage anzubringen. Dieses "ungeordnete Muster" besteht darin, daß die Klebstoffschicht in zahlreiche Einzelpunkte aufgelöst ist, zwischen denen sich eine nicht mit Klebstoff bedeckte Fläche befindet.

Es hat sich gezeigt, daß Klebstoffschichten dieser Art, die praktisch über die gesamte Artikel-Unterseite ausgedehnt sind, technisch außerordentlich schwierig herzustellen sind und deshalb bisher nicht eingesetzt wurden. Tatsächlich konnte man auch bisher eine optimale Haftung des Zellstoffartikels am Kleidungsstück gewährleisten. Was auf die beschriebene Art aber nicht erreicht werden kann, ist das problemlose Abtrennen des Zellstoffartikels vom Kleidungsstück, bei dem keinerlei Klebstoffreste am Kleidungsstück zurückbleiben. Nach Erkenntnissen des Erfinders rührt dies daher, daß die einzelnen Klebstoff-Flecken, welche das erwähnte Muster bilden, immer noch verhältnismäßig große in sich geschlossene Flächen darstellen, die mit einer verhältnismäßig dicken Klebstoffschicht bedeckt sind. Eine Möglichkeit, die Dicke dieser Klebstoffschicht zu reduzieren und damit auch die Klebstoffmenge pro Flächeneinheit herabzusetzen, ist der DE-A 3 128 398 nicht zu entnehmen und auch sonst nicht bekannt.

Der Erfindung liegt die Aufgabe zugrunde, die vorbekannten hygienischen Zellstoffartikel mit Klebeschicht an der Unterseite derart weiterzuentwickeln, daß bei möglichst großflächiger Abdeckung der Unterseite die je Flächeneinheit angewandte Klebstoffmenge weiter reduziert wird, ohne jedoch die Haftung des Zellstoffartikels im Kleidungsstück zu beeinträchtigen.

Zur Lösung dieser Aufgabe wird ein hygienischer Zellstoffartikel vorgeschlagen, der dadurch gekennzeichnet ist, daß die Schmelzkleberhaftschicht in Form von dünnen Fäden in wirrer oder musterförmiger Anordnung auf der Unterseite des Zellstoffartikels abgelegt ist, und daß der Schmelzhaftkleber die die Unterseite bildende Vliesstoff-Umhüllung durchsetzt und diese mit der nächstunteren Schicht des Zellstoffartikels verbindet.

Grundsätzlich ist es zwar bekannt, die Klebeschicht bei hygienischen Zellstoffartikeln aus Schmelzhaftkleber herzustellen. Die vorbekannten Applikationen dieses Klebstoffes sehen aber entweder ganzflächigen oder doch zumindest großflächigen Auftrag vor (beispielsweise in Form von Balken oder Streifen), welche infolge der pro Flächeneinheit angewandten Klebstoffmenge nicht in der Lage sind, die hier vorliegende Aufgabe zu lösen.

Bei der vorbekannten Applikation von Schmelzhaftklebern auf die Unterseite von hygienischen Zellstoffartikeln ist noch zu beachten, daß die je Flächeneinheit anzuwendende Klebstoffmenge eine beträchtliche Wärmemenge auf den Zellstoffartikel überträgt. Diese Wärmemenge ist so groß, daß Beschädigungen der Vliesstoffbahn, welche als Umhüllung der Zellstoffartikel dient und auf welche die Haftkleberschicht aufgebracht werden soll, kaum zu vermeiden sind. Man hilft sich deshalb in der Art, daß die zu applizierende Schmelzhaftklebermenge zunächst auf das Abdeckpapier aufgebracht und von diesem erst auf die Binde übertragen wird. Das Verfahren hat dabei aber den Nachteil, daß als Abdeckpapier verhältnismäßig stark silikonisierte Papier- oder Kunststoffstreifen eingesetzt werden müssen und daß billigere Erzeugnisse, wie beispielsweise Wachspapier, nicht einsetzbar sind. Die erfindungsgemäß vorgeschlagene Weiterentwicklung, bei der der Schmelzhaftkleber in Form von dünnen Fäden wirr oder musterförmig auf der Unterseite des Zellstoffartikels abgelegt wird, gestattet dagegen eine unmittelbare Applikation auf dem hygienischen Zellstoffartikel, so daß die Verwendung des Schmelzhaftklebers auf den Einsatz bestimmter Abdeckstreifen ohne Einfluß bleibt.

Werden Schmelzhaftkleber in der bisher bekannten Weise appliziert, so gelangen sie im verhältnismäßig weit abgekühlten Zustand auf die Unterseite des Zellstoffartikels. Sie sind in diesem Zustand nicht mehr in der Lage, die Unterseite der Vliesstoffumhüllung zu durchsetzen, sondern haften auf der Oberseite an den dort stets vorhandenen, vom eigentlichen Vliesstoff etwas abstehenden feinen Fasern, ohne tatsächlich in die Vliesstoffumhüllung einzudringen. Erfindungsgemäß wird aber vorgeschlagen, den Schmelzhaftkleber so zu applizieren, daß er die die Unterseite bildende Fliesstoffumhüllung durchsetzt und diese mit der nächstunteren Schicht des Zellstoffartikels verbindet, ohne daß ein erheblicher Druck angewendet werden muß, durch den der Artikel verformt würde. Die nächstuntere Schicht ist in aller Regel eine Wäscheschutzfolie, die beispielsweise aus Polyäthylen oder dergl. besteht. Es gibt aber auch hygienische Zellstoffartikel der hier angesprochenen Art, welche keine Wäscheschutzfolie aufweisen und welche leicht luftdurchlässig sind. Bei diesen Artikeln dringt nun der fadenförmig abgelegte Schmelzhaftkleber durch die unterste Vliesstoffschicht hindurch und haftet unmittelbar an der Saugschicht. Ein weiterer Vorteil, der hiermit verbunden ist, besteht darin, daß das so erzeugte wirre oder musterförmige Fadengelege luftdurchlässig ist und folglich die "Atmungsaktivität" dieser Zellstoffartikel nicht behindert.

Bei Produkten mit einer größeren Breite als 60 mm hat es sich als vorteilhaft erwiesen, wenn der Randbereich der Produkt-Unterseite dichter mit Schmelzhaftkleber-Fäden belegt ist als der Mittenbereich. Umgekehrt kann es bei Produkten mit einer kleineren Breite als 60 mm vorteilhaft sein, wenn der sich in Längsrichtung erstreckende Mittenbereich dichter mit Schmelzhaftkleber-Fäden belegt ist als der Randbereich.

Zur Herstellung der beschriebenen Zellstoffartikel wird vorgeschlagen, daß aufgeschmolzener Schmelzhaftkleber aus feinen Düsen fadenförmig ausgepreßt und im noch flüssigen oder plastischen Zustand auf der Unterschicht der hygienischen Zellstoffartikel wirr oder musterförmig abgelegt wird. Das Auspressen von geschmolzenem Schmelzhaftkleber aus feinen Düsen ist grundsätzlich in der Klebetechnik bekannt. Die Anwendung dieser Technik auf hygienische Zellstoffartikel der hier angesprochenen Art, also beispielsweise Damenbinden, Slipeinlagen oder dergl. bringt jedoch die erwähnten nicht vorhersehbaren Vorteile mit sich.

Bei einer besonderen Ausführungsform des Herstellungsverfahrens wird so vorgegangen, daß die Schmelzhaftkleberfäden mit Hilfe eines Heißgasstromes auf einer gasdurchlässigen Unterlage, insbesondere Vliesstoffschicht abgelegt und diese danach zum fertigen Zellstoffartikel weiterverarbeitet wird. Dadurch, daß die Klebstoffschicht zunächst auf die noch freie unverarbeitete Vliesstoffbahn gelegt wird, ist es besonders leicht möglich, daß die heißen Schmelzhaftkleberfäden den Vliesstoff vollständig durchdringen, speziell dann, wenn der den Heißschmelzkleber transportierende Heißluftstrom unterhalb der Vliesstoffbahn abgesaugt wird. Bei der unmittelbar danach erfolgenden Umhüllung der soweit vorgefertigten Saugkörper mit Wäscheschutzfolie und dergl. findet dann die gewünschte Verbindung zwischen der Innenseite des Vliesstoffes mit der nächstunteren Schicht des Saugkörpers statt. Die hier erwähnte besondere Verfahrensvariante findet insbesondere dann Anwendung, wenn die Schmelzhaftkleberfäden auf hygienische Zellstoffartikel mit eingelegter Wäscheschutzfolie aufgebracht werden sollen. Bei Artikeln, die ohne eine solche Folie gefertigt werden, können die Fäden unmittelbar auf der Unterseite des Artikels abgelegt werden, da diese Artikel hinreichend luftdurchlässig sind, um auch hier eine weitgehende oder gar vollständige Durchdringung der Unterschicht (Vliesstoffschicht) zu gewährleisten.

Die Erfindung wird im folgenden anhand der beigefügten Zeichnung näher erläutert. Es stellen dar:

Fig. 1 eine perspektivische Ansicht einer Ausführungsform einer Slipeinlage mit erfindungsgemäß ausgestalteter Klebeschicht;

Fig. 2 einen Querschnitt entlang der Linie II–II der Fig. 1;

Fig. 3 eine schematische Seitenansicht einer Klebstoff-Auftragsstraße, an der das Herstellungsverfahren erläutert wird.

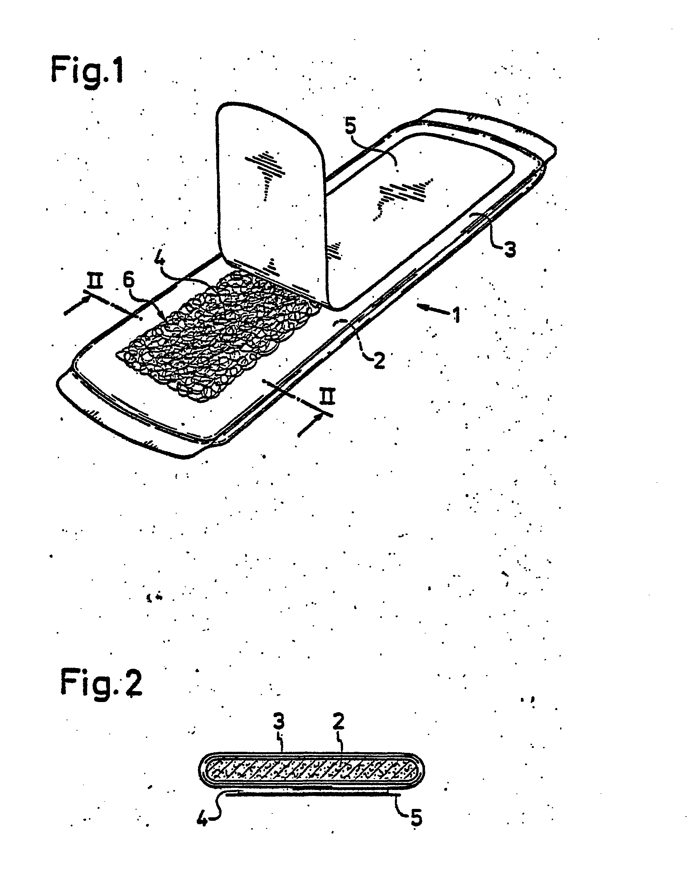

Der in Fig. 1 dargestellte hygienische Zellstoffartikel ist eine Slipeinlage, die als Ganzes mit 1 bezeichnet ist. Sie besteht aus einem Saugkissen 2, einer Vliesstoffumhüllung 3 sowie einer Haftklebeschicht 4, die sich an der Unterseite der Slipeinlage (des Zellstoffartikels) befindet. Die Haftklebeschicht 4 ist bei Nichtgebrauch mit einem schwach haftenden Streifen 5 abgedeckt. Dieser Abdeckstreifen besteht in der Regel aus silikonisiertem Papier oder Kunststoff, er kann aber auch aus Wachspapier oder dergl. hergestellt sein. In Fig. 1 ist der Streifen teilweise von der Klebstoffschicht abgezogen und nach oben geklappt, so daß die darunter liegende Schicht sichtbar wird.

Fig. 1 läßt erkennen, daß die Klebeschicht 4 aus dünnen Fäden 6 aufgebaut ist, die wirr (im dargestellten Beispiel wirr, sonst aber auch musterförmig) auf der Unterseite des Zellstoffartikels abgelegt sind. Die wirre Ablage bringt die erwähnten Vorteile mit sich und sie gestattet es, mit weniger Klebstoff auszukommen als dies bei geschlossenflächiger Ablage möglich wäre. Die Reduzierung der Klebstoffmenge wird hier durch die fadenförmige Ablage möglich, die zudem noch eine Verteilung über eine größere Fläche ermöglicht und die Bildung von jeweils mit Klebstoff unbedeckten Zwischenflächen, durch die die Klebeschicht luftdurchlässig wird.

Fig. 1 läßt desweiteren erkennen, daß die Klebeschicht 4 im Bereich der Längsachse dichter mit Schmelzhaftkleberfäden belegt ist als am Rande. Durch gesteuerte Ablage der aus dünnen Düsen ausgepreßten Fäden ist die Bildung nahezu jeden beliebigen Musters möglich. Es kann so auch umgekehrt eine dichtere Ablage in den Randbereichen und eine spärlichere Ablage im Mittenbereich erzielt werden, wenn dies aus speziellen Gründen einmal gewünscht werden sollte.

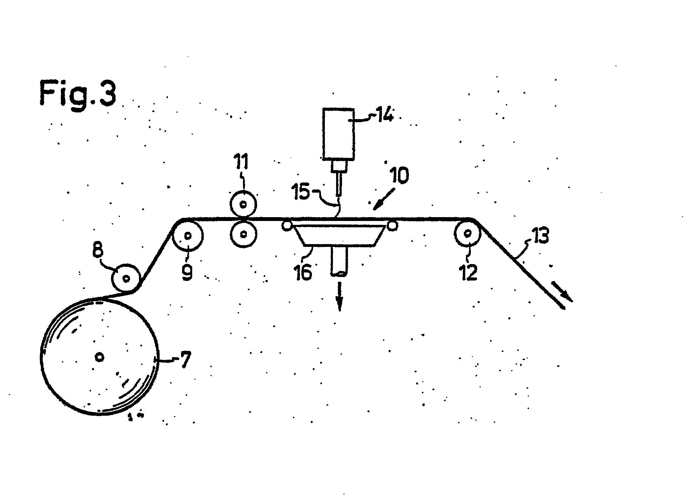

In Fig. 3 ist schematisch ein Aufbau zum Aufbringen von Schmelzhaftkleberfäden auf eine Vliesstoffbahn dargestellt. Die zu bearbeitende Vliesstoffbahn wird in bekannter Weise von einer Rolle 7 abgezogen und mittels Umlenkwalzen 8 und 9 der Behandlungsebene 10 zugeführt. Auf dieser Behandlungsebene kann die Bahn mit Hilfe des Walzenpaares 11 glattgehalten und mit gewünschter Geschwindigkeit angetrieben werden. Am Ende der Behandlungsebene befindet sich die Umlenkwalze 12, über die die Vliesstoffbahn 13 durch nicht dargestellte Mittel abgezogen wird.

In der Behandlungsebene 10 ist wenigstens ein beheizter Schmelzspinnkopf 14 angeordnet. In diesem Spinnkopf befindet sich die auf Spinntemperatur erwärmte geschmolzene Schmelzhaftklebermasse. Durch spezielle Düsenformen ist es möglich, spezielle Klebstoffmuster oder wirr abgelegte Fäden zu erzeugen. Voraussetzung dafür ist ein geeignetes Verhältnis von Klebstoff und Lufttemperatur sowie Klebstoff- und Luftmenge pro Zeiteinheit. Der Spinnkopf kann so angeordnet werden, daß er in zufälliger Folge oder nach vorgegebenem Muster hin und her bewegt werden kann.

Die Schmelzhaftkleberfäden 15 werden vorzugsweise mit Hilfe eines Heißgasstromes auf die Vliesstoffbahn aufgelegt. Der Gasstrom kann dabei durch eine Absaugevorrichtung 16, die unterhalb der Behandlungsebene 12 angeordnet ist, abgesaugt werden. Vorteil dieses Verfahrens ist, daß die noch schmelzflüssig auf die Vliesstoffbahn auftreffenden Fäden die Vliesstoffbahn durchdringen, so daß diese auch an der Unterseite klebend wird, so daß sie sich bei Umhüllung des Saugkissens mit der unteren Schicht dieses Kissens verbindet.

Bezugszeichenliste

1 = Slipeinlage
2 = Saugkissen
3 = Vliesstoff-Umhüllung
4 = Haftklebeschicht
5 = Abdeckstreifen
6 = dünne Fäden
7 = Rolle
8 = Umlenkwalze
9 = Umlenkwalze
10 = Behandlungsebene
11 = Walzenpaar
12 = Umlenkwalze
13 = Vliesstoffbahn
14 = Schmelzspinnkopf
15 = Fäden
16 = Absaugevorrichtung

## Patentansprüche

1. Hygienische Zellstoffartikel mit einem Saugkissen, einer Vliesstoffumhüllung sowie wenigstens einer Schmelzkleber-Haftschicht an der Unterseite, deren Klebstoffschicht teilweise durchbrochen ist, dadurch gekennzeichnet, daß die Schmelzkleber-Haftschicht (4) in Form von dünnen Fäden in wirrer oder musterförmiger Anordnung auf der Unterseite des Zellstoff-Artikels abgelegt ist, und daß der Schmelzhaftkleber die die Unterseite bildende Vliesstoff-Umhüllung durchsetzt und diese mit der nächstunteren Schicht des Zellstoff-Artikels verbindet.

2. Zellstoff-Artikel nach Anspruch 1, dadurch gekennzeichnet, daß bei Produkten mit einer größeren Breite als 60 mm der Randbereich der Produkt-Unterseite dichter mit Schmelzhaftkleber-Fäden belegt ist als der Mittenbereich.

3. Zellstoff-Artikel nach Anspruch 1, dadurch gekennzeichnet, daß bei Produkten mit einer kleineren Breite als 60 mm der in Längsrichtung sich erstreckende Mittenbereich dichter mit Schmelzhaftkleber-Fäden belegt ist als der Randbereich.

4. Verfahren zum Herstellen eines hygienischen Zellstoff-Artikels nach einem der Ansprüche 1 bis 3, dadurch gekennzeichnet, daß zum Aufbringen der Schmelzhaftkleber-Fäden aufgeschmolzener Schmelzhaftkleber aus feinen Düsen fadenförmig ausgepreßt und im flüssigen oder plastischem Zustand auf der Unterschicht des hygienischen Zellstoff-Artikels wirr oder musterförmig abgelegt wird.

5. Verfahren nach Anspruch 4, dadurch gekennzeichnet, daß die Schmelzhaftkleber-Fäden mit Hilfe eines Heißgasstromes auf einer gasdurchlässigen Unterlage, insbesondere Vliesstoff-Schicht, abgelegt und diese danach zum fertigen Zellstoff-Artikel weiterverarbeitet wird.

## Claims

1. Hygienic cellulose article with a suction pad, a fleece material envelope as well as at least one fusible adhesive layer at the underside, the adhesive substance layer of which is at least partially perforated, characterised thereby, that the fusible adhesive layer (4) is deposited in the form of thin threads in irregular or patterned arrangement on the underside of the cellulose article and that the fusible adhesive substance penetrates the fleece material envelope forming the underside and connects this with the next lower layer of the cellulose article.

2. Cellulose article according to claim 13, characterised thereby, that in the case of products of a width greater than 60 millimetres, the rim region of the underside of the product is more densely covered with fusible adhesive substance threads than the middle region.

3. Cellulose article according to claim 1, characterised thereby, that in the case of products of a width smaller than 60 millimetres, the middle region extending in longitudinal direction is more densely covered with fusible adhesive substance threads than the rim region.

4. Method for the production of a hygienic cellulose article according to one of the claims 1 to 3, characterised thereby, that for the application of the fusible adhesive substance threads, molten fusible adhesive substance is pressed in filamentary form out of fine nozzles and deposited irregularly or in the shape of a pattern in liquid or plastic state on the underside of the hygienic cellulose article.

5. Method according to claim 4, characterised thereby, that the fusible adhesive substance threads are deposited with the aid of a current of hot gas on an underlay permeable by gas, in particular a fleece material layer, and this is thereafter processed into the finished cellulose article.

## Revendications

1.- Article hygiénique en cellulose, avec un coussin d'aspiration, une enveloppe en non-tissé, ainsi qu'au moins une couche adhésive en colle à fusion sur la face inférieure, dont la couche de colle est partiellement ajourée, article en cellulose caractérisé en ce que la couche adhésive (4) en colle à fusion est déposée sous la forme de fils fins disposés en désordre ou sous forme de motifs sur la face inférieure de l'article en cellulose, et la colle à fusion traverse l'enveloppe en non-tissé constituant la face inférieure et la relie à la couche inférieure suivante de l'article en cellulose.

2.- Article en cellulose selon la revendication 1, caractérisé en ce que, pour des produits d'une largeur supérieure à 60 mm, le dépôt des fils de colle à fusion est plus dense sur la zone de bordure de la face inférieure du produit que sur la zone médiane.

3.- Article en cellulose selon la revendication 1, caractérisé en ce que pour des produits d'une largeur inférieure à 60 mm, le dépôt des fils de colle à fusion est plus dense sur la zone médiane s'étendant en direction longitudinale que sur la zone de bordure.

4.- Procédé pour fabriquer un article hygiénique en cellulose selon une des revendications 1 à 3, caractérisé en ce que la colle à fusion fondue pour le dépôt des fils de colle à fusion est extraite, sous pression et sous forme de fils, à partir de fines buses, et est déposée, à l'état liquide ou plastique, sur la couche inférieure de l'article hygiénique en cellulose, en désordre ou sous forme de motifs.

5.- Procédé selon la revendication 4, caractérisé en ce que les fils de colle à fusion sont déposés, à l'aide d'un courant de gaz chaud, sur un support perméable aux gaz, notamment une couche de non-tissé, et celle-ci reçoit ensuite un complément de traitement pour terminer la fabrication de l'article en cellulose.

Fig.1

Fig.2

Fig.3